# EUROPEAN PATENT SPECIFICATION

(11) **EP 2 081 509 B1**
(45) Date of publication and mention of the grant of the patent: **23.01.2013**
(21) Application number: 07863431.8
(22) Date of filing: 18.10.2007
(51) Int. Cl.: A61B 17/70

(54) **STRUCTURES FOR CONSTRAINING SPINAL PROCESSES WITH SINGLE CONNECTOR**
STRUKTUREN ZUM HALTEN VON WIRBELFORTSÄTZEN MIT EINEM EINZELNEN VERBINDUNGSELEMENT
STRUCTURES DE CONTRAINTE DE PROCESSUS SPINAUX À CONNECTEUR UNIQUE

(30) Priority: 19.10.2006 US 862085 P
(43) Date of publication of application: 29.07.2009
(73) Proprietor: Simpirica Spine, Inc., San Carlos, CA 94070 (US)
(72) Inventor: ALAMIN, Todd, Woodside, California 94062 (US); BENNETT, Ian, San Francisco, California 94114 (US); FIELDING, Louis, San Carlos, California 94070 (US); CAHILL, Colin, San Francisco, California 94131 (US); KOTHARI, Manish, San Rafael, California 94901 (US)
(74) Representative: Clark, Jane Anne
(86) International application number: PCT/US2007/081815
(87) International publication number: WO 2008/051801

(56) References cited:
- US-A- 5 609 634
- US-A1- 2002 095 154
- US-A1- 2002 147 449
- US-A1- 2004 143 268
- US-A1- 2005 123 581
- US-A1- 2005 216 017
- US-B1- 6 451 019

## Description

### BACKGROUND OF THE INVENTION

1. Field of the Invention. The present invention relates generally to a spinous process constraint structure for restricting spinal flexion in patients having back pain or other spinal conditions.

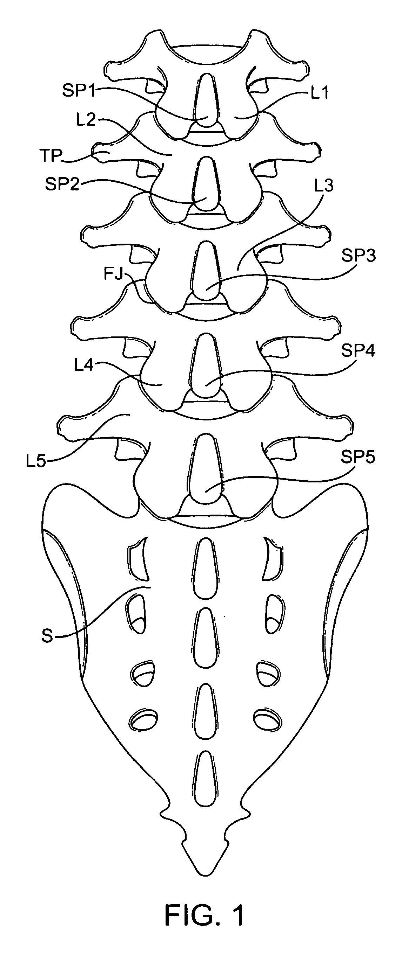

A major source of chronic low back pain is discogenic pain, also known as internal disc disruption. Patients suffering from discogenic pain tend to be young, otherwise healthy individuals who present with pain localized to the back. Discogenic pain usually occurs at the discs located at the L4-L5 or L5-S 1 junctions of the spine (Fig. 1). Pain tends to be exacerbated when patients put their lumbar spines into flexion (i.e. by sitting or bending forward) and relieved when they put their lumbar spines into extension (i.e. arching backwards). Discogenic pain can be quite disabling, and for some patients, can dramatically affect their ability to work and otherwise enjoy their lives.

This pain experienced by patients with discogenic low back pain can be thought of as flexion instability, and is related to flexion instability that is manifested in other conditions. The most prevalent of these is spondylolisthesis, a spinal condition in which abnormal segmental translation is exacerbated by segmental flexion. The device described here should as such also be useful for these other spinal disorders associated with segmental flexion, for which the prevention or control of spinal segmental flexion is desired.

Current treatment alternatives for patients diagnosed with chronic discogenic pain are quite limited. Many patients follow a conservative treatment path, such as physical therapy, massage, anti-inflammatory and analgesic medications, muscle relaxants, and epidural steroid injections, but typically continue to suffer with a significant degree of pain. Other patients elect to undergo spinal fusion surgery, which commonly requires discectomy (removal of the disk) together with fusion of adjacent vertebra. Fusion is not usually recommended for discogenic pain because it is irreversible, costly, associated with high morbidity, and of questionable effectiveness. Despite its drawbacks, however, spinal fusion for discogenic pain remains common due to the lack of viable alternatives.

Recently, a less invasive and potentially more effective treatment for discogenic pain has been proposed. A spinal implant has been designed which inhibits spinal flexion while allowing substantially unrestricted spinal extension. The implant is placed over one or more adjacent pairs of spinal processes and provides an elastic restraint to the spreading apart of the spinal processes which occurs during flexion. Such devices and methods for their use are described in U.S. Patent Application 2005/0216 017A1, published on September 29, 2005, and having common inventors with the present application.

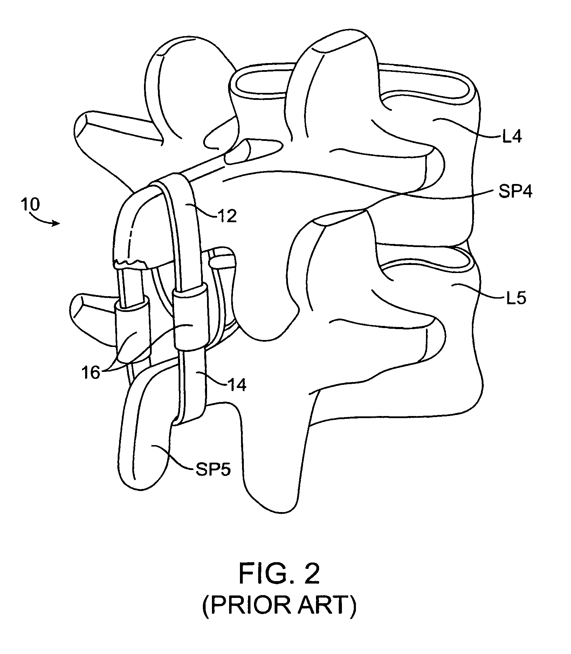

As illustrated in Fig. 2, an implant 10 as described in the '017 application, typically comprises an upper strap component 12 and a lower strap component 14 joined by a pair of compliant members 16. The upper strap 12 is shown disposed over the top of the spinous process SP4 of L4 while the lower strap 14 is shown extending over the bottom of the spinous process SP5 of L5. The compliant member 16 will typically include an internal element, such as a spring of rubber block, which is attached to the straps 12 and 14 in such a way that the straps may be "elastically" or "compliantly" pulled apart as the spinous processes SP4 and SP5 move apart during flexion. In this way, the implant provides an elastic tension on the spinal processes which provides a force that resists flexion. The force increases as the processes move further apart. Usually, the straps themselves will be essentially non-compliant so that the degree of elasticity or compliance may be controlled and provided solely by the compliance members 16.

Ideally, the compliance members 16 will remain horizontally aligned and spaced generally between the spinous processes SP4 and SP5. In some instances, however, the desired symmetry may be lost if the implant structure 10 becomes circumferentially displaced about the spinous processes SP4 and SP5. Such displacement can affect the ability of the implant to provide a uniform, symmetric elastic force to inhibit flexion of the spinous processes of a spinal segment in accordance with the desired treatment. Also, the symmetric designs illustrated in Fig. 2 can be difficult to deliver from the side which would be a preferred approach in percutaneous delivery techniques.

For these reasons, it would be desirable to provide improved spinal implants and methods for their use in inhibiting flexion in patients suffering from discogenic pain. It would be particularly desirable if the improved devices would provide the desired elastic forces to the spinous processes with minimal risk of displacement or loss of symmetry of the device over time. It would be further desirable if the designs facilitated percutaneous delivery from the side and other approaches. Additionally, it would be advantageous if the implants and implantation methods could be performed with minimum tissue disruption via percutaneous and open surgical procedures. At least some of these objectives will be met by the invention as described hereinbelow.

2. Description of the Background Art. US 2005/0216017A1 has been described above. Other patents and published applications of interest include: U.S. Patent Nos. 4,966,600; 5,011,494;5,092,866; 5,116,340; 5,282,863; 5,395,374; 5,415,658; 5,415,661; 5,449,361; 5,456,722; 5,462,542; 5,496,318; 5,540,698; 5,609,634; 5,645,599; 5,725,582; 5,902,305; Re. 36,221; 5,928,232; 5,935,133; 5,964,769; 5,989,256; 6,053,921; 6,312,431; 6,364,883; 6,378,289; 6,391,030; 6,468,309; 6,436,099; 6,451,019; 6,582,433; 6,605,091; 6,626,944; 6,629,975; 6,652,527; 6,652,585; 6,656,185; 6,669,729; 6,682,533; 6,689,140; 6,712,819; 6,689,168; 6,695,852; 6,716,245; 6,761,720; 6,835,205; Published U.S. Patent Application Nos. US 2002/0151978; US 2004/0024458; US 2004/0106995; US 2004/0116927; US 2004/0117017; US 2004/0127989; US 2004/0172132; US 2005/0033435; US 2005/0049708; US 2006/0069447; Published PCT Application Nos. WO 01/28442 A1; WO 02/03882 A2; WO 02/051326 A1; WO 02/071960 A1; WO 03/045262 Al; WO 2004/052246 A1 WO 2004/073532 A1; and Published Foreign Application Nos. EP 0322334 A1; and FR 2 681 525 A1.

US 6451019 describes a supplemental spine fixation device which includes a guide and spacer for distancing apart adjacent spinous processes. The device has hook members which hook about the first and second spinous processes. With the spinous processes distracted and the hook members about the spinous processes, the hook members can be rigidly secured to a hub in order to rigidly affix the spinous processes about the spacer. With such an arrangement, additional force is placed on vertebral bodies in order to encourage fusion.

US 2005/0123581 describes a compressed high density fibrous polymer suitable for implant.

US 2002/0095154 describes a dynamic bias device to apply a bias force to adjacent vertebrae.

US 5609634 describes a prosthesis including a flexible ligament with a first narrowed portion and a second wider portion which may be passed round a spinous processes of two adjacent vertebrae in a figure of eight pattern.

FR 2828398 describes a vertebrae stabilizing assembly comprising a rear damping implant configured to be laterally placed at the blade-spinal junction of two vertebrae and at least a damping interbody implant configured to be inserted between the vertical end plates of the two treated vertebrae.

EP 0743045 describes a device for osteosynthesis of the kind prepared of a material which exhibits form-memory effect.

US 2004/0117017 describes a self locking fixable intervertebral implant.

WO 2005/110258 describes an intervertebral implant which is fixedly placed between spinous processes to maintain the predetermined space between the spinous processes.

FR 2884136 describes a surgical intervertebral implant forming a swivel joint.

FR 2717675 describes a shock-absorbing spacer block for location between adjacent vertebrae.

FR 2722980 describes an interspinal vertebral implant comprising a substantially U-shaped body having an elastic flexibility in the area of its central portion, and two pairs of brackets projecting from the outer face of the two branches of the body, these brackets defining stirrups for receiving the spinous processes of the vertebrae and comprising means for permitting them to be fixed to the processes.

US 2005/0203624 describes an interspinous implant having a general horseshoe shape, a cushion element within the horseshoe and a porous coating on its outer surface.

FR 2693364 describes an intervertebral prosthesis including a flexible ligament with a first narrow thinner portion and a second wider portion forming an extension of, and having a greater width and thickness than, the first portion, which portion may be passed around the spinous processes of two adjacent vertebrae in a figure eight pattern, whereas the second portion circles around the processes. A semi-flexible interprocess block with two pairs of through-channels having differing cross-sections receives the respective portions of the ligament, whose ends are joined.

WO 2005/037150 describes a non-rigid system for stabilising displaced bony members including a flexible unit of tethering material coupled to the disposed bony members so as to restore a desired shape, curvature or relationship between the bony members without excessively limiting mobility of the bony members.

### BRIEF SUMMARY OF THE INVENTION

The present invention is set out in the appended Claims. Described herein are spinal implants and methods for restricting flexion of spinal segments for the treatment of discogenic pain and other spinal conditions, such as spondylolisthesis, where a physician may desire to control segmental flexion. Described systems include spinous process constraint structures comprising a first attachment element adapted to be placed over a first spinous process, a second attachment element adapted to be placed over a second spinous process, and a single connector joining the first attachment element and the second attachment element. By "single connector," it is meant that the connector joins a single point or location on the first attachment element to a single point or location on the second attachment element. In contrast, the prior connectors shown in Fig. 2, for example, provide a pair of connection points and two connectors for joining the upper component 12 to the lower strap component 14. Use of the single connector for joining the first and second attachment elements reduces the likelihood that the attachment members will become displaced such that the desired symmetric attachment geometry becomes asymmetric. A single connector also reduces the need to balance the elastic forces being applied to the opposite sides of the spinous processes. The single connector will also simplify alignment of the implant during implantation, thus simplifying percutaneous implantation and potentially minimizing tissue disruption in both percutaneous and other implantation protocols.

The first and second attachment elements may have similar or different geometries. Exemplary geometries include open hook structures which may be placed about the spinous processes and which have a single attachment point for connection to the single connector. The attachment elements may also be loop structures which fully circumscribe the spinous process, where the loop is provided with a single connection point for connection to the single connector. Often, the attachment elements will be placed over the spinous process without further attachment. In other instances, however, it may be desirable to provide a secondary attachment to the spinous process, such as staples, pins, sutures, adhesives, energy-mediated attachments (such as laser welding), or the like. In some instances, one of the two attachment elements may be adhered to the adjacent spinous process while the other of the attachment elements may be simply placed over the adjacent spinous process without adherence.

The constraint structures of the present invention may comprise separate components which are joined or connectable together. For example, each of the first attachment element, the second attachment element, and the single connector may be formed separately and interconnected by conventional techniques, such as screwing, welding, linking with male and female attachment members, strapping, soldering, or any other such fastening technique. In other instances, any two or more of the components of the constraints of the present invention may be integrally or monolithically formed from a common structural member. For example, a pair of hook-like elements may be integrally formed with an - intermediate connector by forming the components from a single rod, wire, cable, polymer substrate, or the like.

The spinous process constraints of the present invention may further comprise a compliance member disposed within or as part of the single connector. The compliance member may have any structure which provides for the desired elasticity in the connector to permit the first and second attachment elements to spread apart as the spinal segment undergoes flexion. Suitable compliance members are described in published U.S. Application No. 2005/0216017 A1, which has been previously incorporated herein by reference.

In other embodiments, the single connector may comprise an elastomeric body which is disposed between the first and second attachment elements. In some instances, the elastomeric body may be positionable over the supraspinous ligament, and in certain of those cases such elastomeric bodies may be adapted to be sutured or otherwise attached to the supraspinous ligament.

Methods are described herein for restricting flexion of a spinal segment comprise positioning a first attachment element on a first spinous process and positioning a second attachment element on a second spinous process, wherein the attachment members are joined by a single connector. The attachment members may be positioned in an open surgical procedure through the supraspinous ligament or may be percutaneously implanted, optionally from a single sided posterior approach avoiding the need to penetrate the supraspinous ligament. In a specific example, the elements are joined with an elastic member, where the elastic member is preferably positioned over the supraspinous ligament. In particular examples, the methods further comprise attaching the elastic member to the supraspinous ligament, for example by suturing. Usually, the methods further comprise penetrating the supraspinous ligament to permit passage of the attachment element(s) and/or the elastic member therethrough. Still further optionally, the attachment members may be attached to the spinous processes, typically by stapling or any of the other attachment modalities described above.

### BRIEF DESCRIPTION OF THE DRAWINGS

Fig. 1 is a schematic diagram illustrating the lumbar region of the spine including the spinous processes (SP), facet joints (FJ), lamina (L), transverse processes (TP), and sacrum (S).

Fig. 2 illustrates a spinal implant of the type described in US 2005/0216017A1.

Fig. 3 illustrates an exemplary embodiment of a spinous process constraint structure constructed in accordance with the principles of the present invention.

Figs. 4-11 are schematic illustrations of additional exemplary embodiments of the spinous process constraint structures of the present invention, where the adjacent spinous processes are shown in section.

Fig. 12 illustrates a further alternative embodiment of a spinal constraint structure of the present invention shown with a first attachment member placed over the spinous process of L5 and a lower attachment member attached to the sacrum.

Figs. 13 and 14 illustrate another embodiment of a spinous process constraint structure providing background to the present invention where the attachment members are placed over adjacent spinous processes with the single connector passing through and over the supraspinous ligament.

### DETAILED DESCRIPTION OF THE INVENTION

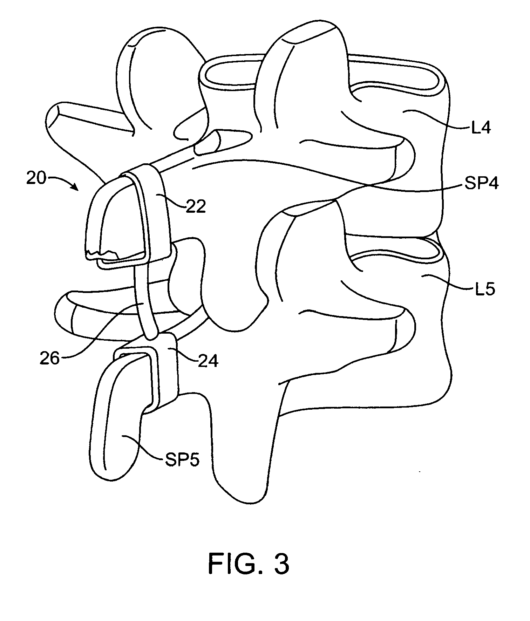

Referring now to Fig. 3, a spinous process constraint structure 20 constructed in accordance with the principles of the present invention comprises a first or upper attachment member 22 and a second or lower attachment member 24. The first and second attachment members are connected by a single connector 26, shown in the form of an elastic rod or cable. Usually, the attachment members 22 and 24 is non-distensible, and is firmly placed over the spinous processes, shown as the spinous process SP4 of L4 and the spinous process SP5 of L5. The connector 26 is elastically distensible so that it comprises an elastic constraining force as a spinal segment undergoes flexion which causes the spinous processes SP4 and SP5 to spread vertically apart. While being elastic in tension, the single connector 26 has a very low column strength so that it exerts very little force on the spinous processes SP4 and SP5 when the spinal segment is in extension and the processes move vertically toward one another. As used herein, the phrase "spinal segment" is synonymous with the phrase "functional spinal unit (FSU)" and intended to mean the smallest physiological motion unit of the spine that exhibits biomechanical characteristics similar to those of the entire spine. A spinal segment or FSU consists of two adjacent vertebrae, the intervertebral disc and all adjoining ligaments between them and excludes other connecting tissues such as muscles. The three-joint complex that results is sometimes referred to as the "articular triad." Another term for the FSU is spinal motion segment These definitions are taken from White AA, Panjabi MM. (1990), Clinical Biomechanics of the Spine, Philadelphia, JB Lippincott.

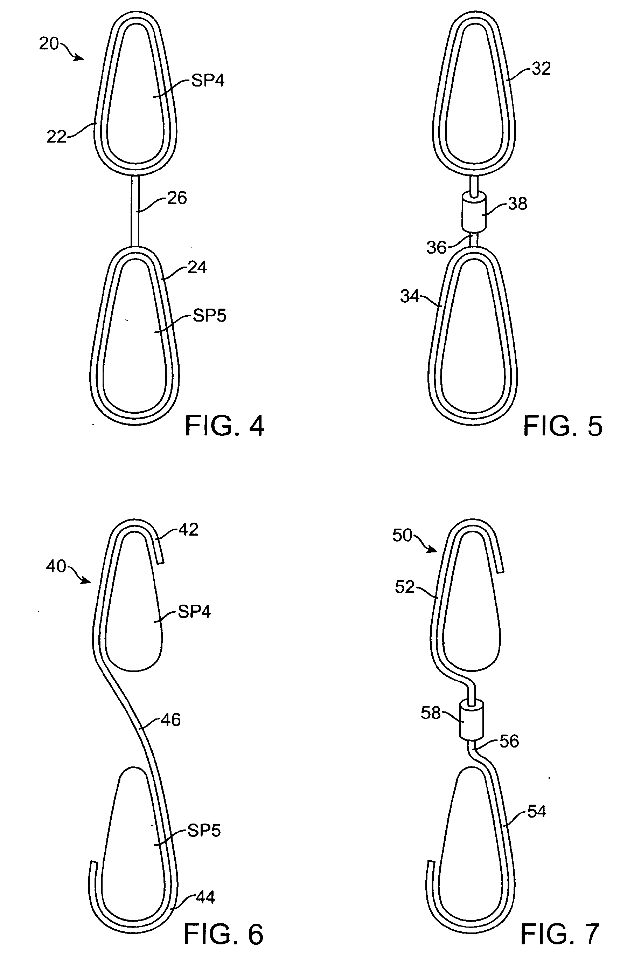

The first and second attachment members 22 and 24 may be wrapped around the associated spinous process SP4 and SP5 without further adherence or fastening. In some cases, however, it may be desirable to staple, suture, glue, or otherwise attach the attachment members to the underlying spinous process. It will also be appreciated that in many instances the attachment members may have a seam or closure which allows them to be wrapped around the spinous process and closed *in situ* thereover during an implantation procedure. It will be further appreciated that the single connector 26 may be preattached to either or both of the attachment members 22 and 24. In other instances, however, it may be desirable to attach the connector 26 to either or both of the attachment members 22 and 24 during the implantation procedure in order to permit the length of the connector to be adjusted. In particular, it will be desirable that the length of the connector 26 be selected so that the connector is generally fully extended but not under significant tension when the spinal segment is in its neutral (non-flexion and non-extension) condition. In such cases, the connector 26 will begin to apply tension on the spinous processes 22 and 24 as soon as they begin to undergo flexion while collapsing and applying no force on the spinous processes as they undergo extension. Fig. 4 is a schematic cross-sectional view of the spinous process constraint structure 20 of Fig. 3.

Fig. 5 illustrates an alternative spinous process constraint structure 30 having first and second attachment members 32 and 34, similar to those described in connection with Figs. 3 and 4, and joined by a single connector 36 having a compliance member 38. In this embodiment, the single connector 36 is formed from a non-distensible material where the desired elasticity is provided by the compliance member 38.

Referring now to Fig. 6, a spinous process constraint structure 40 having a first or upper hook-like attachment member 42 and a second or lower hook-like attachment member 44 is illustrated. The first and second attachment members 42 and 44 are connected by a single contiguous or integral connector 46, which is transversely oriented in the space between the upper spinous process SP4 and the lower spinous process SP5. The constraint structure 40 may be formed from a spring-like metal, such as spring steel or nickel-titanium alloy, or alternatively may be formed from an elastomeric polymer. In some instances, the hook-like attachment members could be reinforced or otherwise modified to be substantially non-compliant, while the connector 46 could be modified to enhance its elasticity, for example having a serpentine or coil spring structure.

Referring now to Fig. 7, a further spinous process constraint system 50 comprises upper and lower hook-like attachment members 52 and 54 joined by a single connector 56. The upper and lower attachment members 52 and 54 as well as the connector section 56 may be formed from metal or polymer and will typically be non-distensible. The desired elasticity between the attachment members is provided by a compliance member 58.

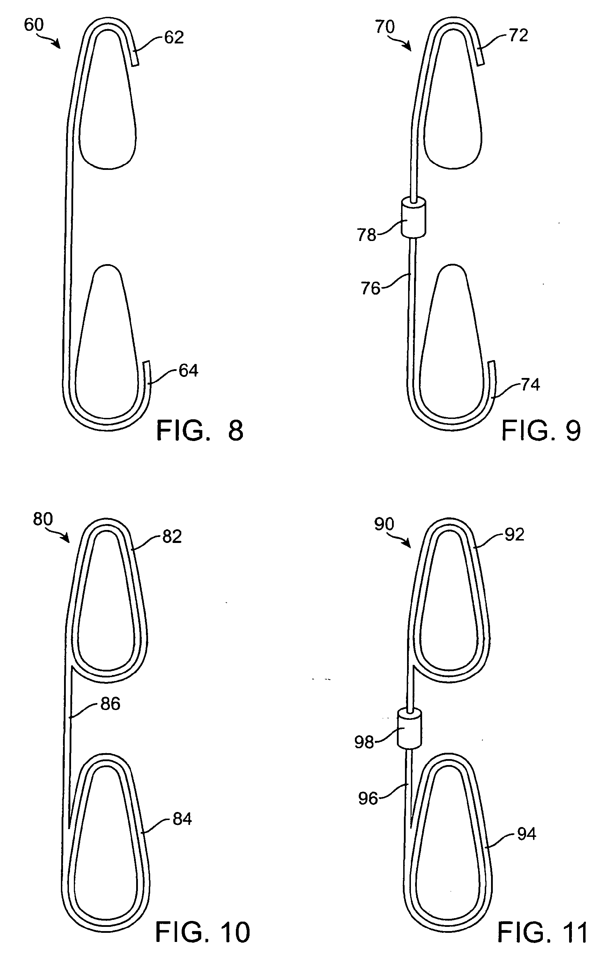

Referring now to Fig. 8, yet another spinous process constraint system 60 comprises first and second hook-like attachment members 62 and 64. Instead of being connected in an S-shaped pattern, as shown in Fig. 6, the hook members 62 and 64 are connected in a C-shaped pattern, as shown in Fig. 8. Other aspects of the constraint system 60 may be similar to those described with respect to constraint 40 of Fig. 6.

Similarly, as shown in Fig. 9, a spinous process constraint system 70 comprises first and second hook-like attachment members 72 and 74 arranged in C-shaped pattern, generally as shown in Fig. 8, further comprises compliance member 78 attached to the single connector 76 (which is preferably non-compliant). Other aspects of the system may be generally as described in connection with the constraint structure 50 of Fig. 7.

In still another embodiment, a spinous process constraint system 80, as shown in Fig. 10, comprises a loop or encircling first attachment member 82 and a loop or encircling second attachment member 84. The attachment members 82 and 84 are joined by a connector 86 which, instead of being attached at the center of the attachment members, is attached laterally to one side. It will be appreciated that the connector 86 could just as well have been attached laterally on the opposite side.

Referring now to Fig. 11, spinous process constraint system 90 comprises upper and lower attachment members 92 and 94 which are similar to those described with respect to constraint structure 80 of Fig. 10. A single connector 96 is typically formed from a non-distensible material, and the desired elasticity is provided by a compliance member 98 provided along the length of the single connector 96.

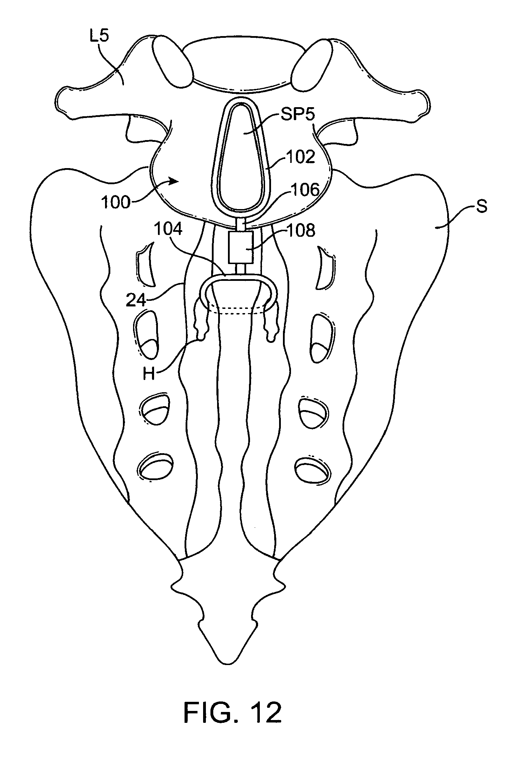

As described thus far, spinous process constraint systems have been intended to be placed on adjacent spinous processes. It will be appreciated that the constraint systems could be placed on spinous processes which are non-adjacent; e.g., separated by one or more additional spinous processes. It will be further appreciated that the spinous process constraint systems could be attached at a first or upper end to the spinous process SP5 of L5 and at a second or lower end to the sacrum S, as shown in Fig. 12. As the sacrum will often not include a process or other structure sufficient for attachment, when attachment member as described previously, spinous process constraint system 100 may include a first or upper attachment member 102 similar to any of those described previously, and a second or lower attachment member 104 which is modified to attach to the sacrum, e.g., by looping through a hole H formed in the structure of the sacrum. Other attachment members suitable for attaching to the sacrum are described in copending Application No. 111827,980, filed on July 13, 2007, (Publication no. US2008 0009866). A single connector 106 is provided between the upper and lower attachment members 102 and 104, optionally including a compliance member 108 to provide the desired elasticity.

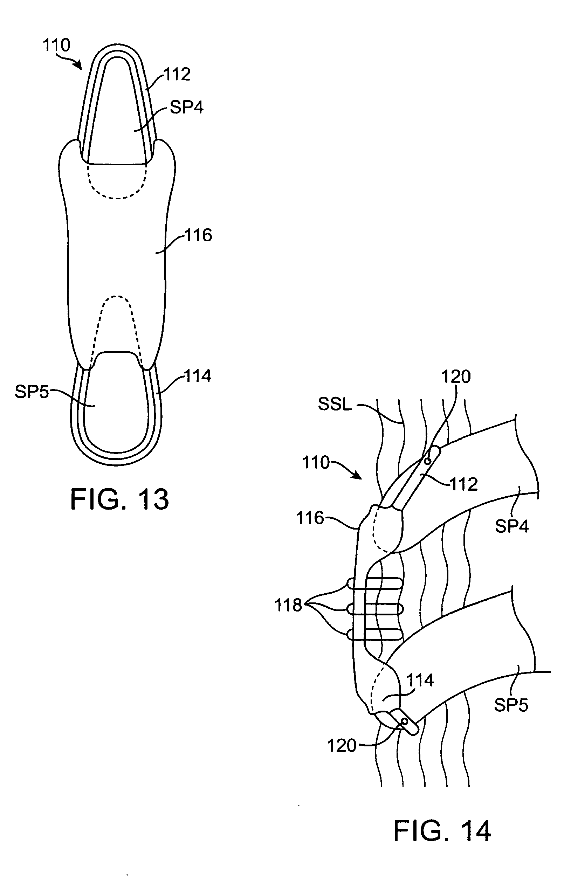

Referring now to Figs. 13 and 14, another spinous process constraint system, not claimed, and method, not claimed, for its implementation are described as background to the present invention. The spinous processes constraint system 110 includes a first or upper attachment member 112 and a second or lower attachment member 114. The upper and lower attachment members are joined by an elastic component, typically an elastomeric body 116 which is configured to be placed over the surface of the supraspinous ligament SSL, as shown in Fig. 14. The advantage of the constraint structure 110 is that it will minimally disrupt the structure of the supraspinous ligament, typically requiring only minor penetrations to allow the placement of the attachment members 112 and 114. Optionally, the elastomeric body 116 may be attached to the supraspinous ligament SSL, for example by sutures 118, or adhesives, staples, or by other conventional attachment means. Similarly, because the elastomeric body 116 will be exerting a rearward force on the attachment members 112 and 114, it will typically be desirable to staple, pin, suture, glue, or otherwise attach the attachment members to the spinous processes SP4 and SP5. While pins 120 are shown, it will be appreciated that any of the other attachment means could also be used.

While the above is a complete description of the preferred embodiments of the invention, various alternatives, modifications, and equivalents may be used. Therefore, the above description should not be taken as limiting the scope of the invention which is defined by the appended claims.

## Claims

1. A spinous process constraint structure (20, 30, 40, 50, 60, 70, 80, 90) comprising:
a first attachment element (22, 32, 42, 52, 62, 72, 82, 92) adapted to be placed over a first spinous process (SP4) of a spinal segment;
a second attachment element (24, 34, 44, 54, 64, 74, 84, 94) adapted to be placed over a second spinous process (SP5) of the spinal segment;
and
a single connector (26, 36, 46, 56, 76, 86, 96) joining the first attachment element and the second attachment element, wherein the single connector (26, 46, 86) is elastically distensible or the single connector (36, 56, 76, 96) is formed from a non-distensible material and has a compliance member (38, 58, 78, 98), and wherein the compliance member provides a desired elasticity,
**characterized in that** the single connector applies no force to the spinous processes as the spinal segment undergoes extension.

2. A spinous process constraint structure as in claim 1, wherein the first and second attachment elements (42, 44, 52, 54, 62, 64, 72, 74) are open hook structures.

3. A spinous process constraint structure as in claim 2, wherein the connector is a transverse element (46, 56) joining the hooks in an S-pattern.

4. A spinous process constraint structure as in claim 3, wherein the structure (40) comprises a continuous metal component shaped into the S-pattern.

5. A spinous process constraint structure as in claim 3, wherein the structure (40) comprises a continuous polymeric structure shaped into the S-pattern.

6. A spinous process constraint structure as in claim 2, wherein the connector is an axial member (76) joining the hooks in a C-pattern.

7. A spinous process constraint structure as in claim 6, wherein the axial member (76) comprises a compliance member (78).

8. A spinous process constraint structure as in any one of the preceding claims, wherein the first and second attachment elements (22, 24, 32, 34, 82, 84, 92, 94) are loop structures which fully circumscribe the spinous process.

9. A spinous process constraint structure as in claim 8, wherein the single connector comprises a transverse element positioned transversely through the space between the spinous processes.

10. A spinous process constraint structure as in claim 8, wherein the single connector comprises an axial member (86, 96) positioned to lie parallel to the sides of the spinous processes.

11. A spinous process constraint structure as in claim 10, further comprising at least one compliance member (98) on the axial member (96).

12. A spinous process constraint structure as in claim 8, wherein the single connector (26) comprises an elastomeric body positionable over the supraspinous ligament.

13. A spinous process constraint structure as in claim 9, wherein the elastomeric body is adapted to be sutured to the suprapinous ligament.

14. A spinous process constraint structure as in claim 1, wherein the single connector (26) is elastically distensible and is in the form of an elastic rod or cable.

## Patentansprüche

1. Dornfortsatzeinschränkungsstruktur (20, 30, 40, 50, 60, 70, 80, 90), Folgendes umfassend:
ein erstes Befestigungselement (22, 32, 42, 52, 62, 72, 82, 92), das dazu angepasst ist, über einen ersten Dornfortsatz (SP4) eines Wirbelsäulensegments gelegt zu werden;
ein zweites Befestigungselement (24, 34, 44, 54, 64, 74, 84, 94), das dazu angepasst ist, über einen zweiten Dornfortsatz (SP5) des Wirbelsäulensegments gelegt zu werden;
und
einen einzelnen Verbinder (26, 36, 46, 56, 76, 86, 96), der das erste Befestigungselement und das zweite Befestigungselement verbindet, wobei der einzelne Verbinder (26, 46, 86) elastisch dehnbar ist, oder der einzelne Verbinder (36, 56, 76, 96) aus einem nicht dehnbaren Material hergestellt ist und über ein Nachgiebigkeitsteil (38, 58, 78, 98) verfügt, und wobei das Nachgiebigkeitsteil eine gewünschte Elastizität bereitstellt,
**dadurch gekennzeichnet, dass** der einzelne Verbinder keine Kraft an die Dornfortsätze anlegt, wenn das Wirbelsäulensegment eine Extension erfährt.

2. Dornfortsatzeinschränkungsstruktur nach Anspruch 1, wobei es sich bei dem ersten und zweiten Befestigungselement (42, 44, 52, 54, 62, 64, 72, 74) um offene Hakenstrukturen handelt.

3. Dornfortsatzeinschränkungsstruktur nach Anspruch 2, wobei es sich bei dem Verbinder um ein Querelement (46, 56) handelt, das die Haken in einem S-Muster verbindet.

4. Dornfortsatzeinschränkungsstruktur nach Anspruch 3, wobei die Struktur (40) einen durchgehenden Metallbestandteil aufweist, der zu dem S-Muster geformt ist.

5. Dornfortsatzeinschränkungsstruktur nach Anspruch 3, wobei die Struktur (40) eine durchgehende Polymerstruktur umfasst, die zu dem S-Muster geformt ist.

6. Dornfortsatzeinschränkungsstruktur nach Anspruch 2, wobei es sich bei dem Verbinder um ein axiales Teil (76) handelt, das die Haken in einem C-Muster verbindet.

7. Dornfortsatzeinschränkungsstruktur nach Anspruch 6, wobei das axiale Teil (76) ein Nachgiebigkeitsteil (78) aufweist.

8. Dornfortsatzeinschränkungsstruktur nach einem der vorhergehenden Ansprüche, wobei es sich bei dem ersten und zweiten Befestigungselement (22, 24, 32, 34, 82, 84, 92, 94) um Schlaufenstrukturen handelt, die um den Dornfortsatz vollständig herumlaufen.

9. Dornfortsatzeinschränkungsstruktur nach Anspruch 8, wobei der einzelne Verbinder ein Querelement aufweist, das quer durch den Raum zwischen den Dornfortsätzen positioniert ist.

10. Dornfortsatzeinschränkungsstruktur nach Anspruch 8, wobei der einzelne Verbinder ein axiales Teil (86, 96) aufweist, das so positioniert ist, dass es parallel zu den Seiten der Dornfortsätze liegt.

11. Dornfortsatzeinschränkungsstruktur nach Anspruch 10, darüber hinaus mindestens ein Nachgiebigkeitsteil (98) an dem axialen Teil (96) umfassend.

12. Dornfortsatzeinschränkungsstruktur nach Anspruch 8, wobei der einzelne Verbinder (26) einen Elastomerkörper aufweist, der über dem Ligamentum supraspinale positioniert werden kann.

13. Dornfortsatzeinschränkungsstruktur nach Anspruch 9, wobei der Elastomerkörper dazu angepasst ist, mit dem Ligamentum supraspinale vernäht zu werden.

14. Dornfortsatzeinschränkungsstruktur nach Anspruch 1, wobei der einzelne Verbinder (26) elastisch dehnbar ist und in der Form eines elastischen Stabs oder Seils vorliegt.

## Revendications

1. Structure de contrainte d'apophyse épineuse (20, 30, 40, 50, 60, 70, 80, 90) comprenant:
un premier élément de fixation (22, 32, 42, 52, 62, 72, 82, 92) adapté pour être placé sur une première apophyse épineuse (SP4) d'un segment rachidien,
un second élément de fixation (24, 34, 44, 54, 64, 74, 84, 94) adapté pour être placé sur une seconde apophyse épineuse (SP5) du segment rachidien,
et
un unique connecteur (26, 36, 46, 56, 76, 86, 96) reliant le premier élément de fixation et le second élément de fixation, dans lequel l'unique connecteur (26, 46, 86) est dilatable de manière élastique ou l'unique connecteur (36, 56, 76, 96) est formé à partir d'un matériau non dilatable et dispose d'un élément de conformité (38, 58, 78, 98), et dans lequel l'élément de conformité fournit une élasticité voulue,
**caractérisée en ce que** l'unique connecteur n'applique aucune force sur les apophyses épineuses lorsqu'une extension du segment rachidien se produit.

2. Structure de contrainte d'apophyse épineuse selon la revendication 1, dans laquelle les premier et second éléments de fixation (42, 44, 52, 54, 62, 64, 72, 74) sont des structures ouvertes à crochets.

3. Structure de contrainte d'apophyse épineuse selon la revendication 2, dans laquelle le connecteur est un élément transversal (46, 56) reliant les crochets dans un motif en forme de S.

4. Structure de contrainte d'apophyse épineuse selon la revendication 3, dans laquelle la structure (40) comprend un composant métallique continu façonné dans le motif en forme de S.

5. Structure de contrainte d'apophyse épineuse selon la revendication 3, dans laquelle la structure (40) comprend une structure polymère continue façonnée dans le motif en forme de S.

6. Structure de contrainte d'apophyse épineuse selon la revendication 2, dans laquelle le connecteur est un élément axial (76) reliant les crochets dans un motif en forme de C.

7. Structure de contrainte d'apophyse épineuse selon la revendication 6, dans laquelle l'élément axial (76) comprend un élément de conformité (78).

8. Structure de contrainte d'apophyse épineuse selon l'une quelconque des revendications précédentes, dans laquelle les premier et second éléments de fixation (22, 24, 32, 34, 82, 84, 94) sont des structures en boucle qui circonscrivent entièrement l'apophyse épineuse.

9. Structure de contrainte d'apophyse épineuse selon revendication 8, dans laquelle l'unique connecteur comprend un élément transversal positionné transversalement à travers l'espace entre les apophyses épineuses.

10. Structure de contrainte d'apophyse épineuse selon la revendication 8, dans laquelle l'unique connecteur comprend un élément axial (86, 96) positionné pour reposer parallèlement aux côtés des apophyses épineuses.

11. Structure de contrainte d'apophyse épineuse selon la revendication 10, comprenant en outre au moins un élément de conformité (98) sur l'élément axial (96).

12. Structure de contrainte d'apophyse épineuse selon la revendication 8, dans laquelle l'unique connecteur (26) comprend un corps élastomère positionnable sur le ligament supra-épineux.

13. Structure de contrainte d'apophyse épineuse selon la revendication 9, dans laquelle le corps élastomère est adapté pour être suturé au ligament supra-épineux.

14. Structure de contrainte d'apophyse épineuse selon la revendication 1, dans laquelle l'unique connecteur (26) est dilatable élastiquement et se présente sous la forme d'une tige ou d'un câble élastique.
